# FASCICULE DE BREVET EUROPEEN

(11) **EP 1 776 981 B1**
(45) Date de publication et mention de la délivrance du brevet: **11.02.2009**
(21) Numéro de dépôt: 06291619.2
(22) Date de dépôt: 19.10.2006
(51) Int. Cl.: A61N 1/375

(54) **Dispositif médical implantable actif à télèmétrie RF pourvu d'électrodes de recueil d'ECG subcutané**
Aktive implantierbare medizinische Vorrichtung mit RF Telemetrie ausgestatteter mit unterkutanen EKG Sammlungselektroden
Active implantable medical device with RF telemetry provided with subcutaneous ECG collection electrodes

(30) Priorité: 21.10.2005 FR 0510738
(43) Date de publication de la demande: 25.04.2007
(73) Titulaire: ELA MEDICAL, 92541 Montrouge (FR)
(72) Inventeur: Dal Molin, Renzo, 92320 Châtillon (FR); Bourguiba, Anissa, 75014 Paris (FR)
(74) Mandataire: Dupuis-Latour, Dominique

(56) Documents cités:
- US-A- 5 331 966
- US-A- 5 861 019
- US-A- 6 144 879
- US-A1- 2004 015 199
- US-A1- 2004 215 280
- US-A1- 2006 095 083
- US-B1- 6 631 290

## Description

L'invention concerne les "dispositifs médicaux implantables actifs" tels que définis par la directive 90/385/CEE du 20 juin 1990 du Conseil des communautés européennes, plus précisément les dispositifs permettant de surveiller en continu le rythme cardiaque et délivrer éventuellement au coeur des impulsions électriques de stimulation, de resynchronisation, de cardioversion et/ou de défibrillation en cas de trouble du rythme détecté par l'appareil.

L'invention concerne plus précisément ceux de ces dispositifs qui sont pourvus de moyens de recueil de signaux d'électrocardiogramme (ECG) subcutané, c'est-à-dire dont le boîtier implanté est pourvu d'électrodes permettant de recueillir directement ces signaux depuis l'intérieur du corps, sans recourir à des électrodes de surface appliquées sur la peau, comme dans le cas des enregistreurs ECG traditionnels.

Le recueil des signaux d'ECG subcutané est à distinguer du recueil des signaux d'électrogramme (EGM), qui requiert l'utilisation de sondes endocavitaires implantées dans le myocarde pour mesurer le potentiel de dépolarisation, principalement aux fins de pilotage d'un stimulateur cardiaque. Les signaux ECG, quant à eux, sont destinés à être enregistrés sur une longue période pour être ensuite traités et analysés au moyen d'algorithmes de reconstruction permettant d'apprécier l'état clinique du patient et diagnostiquer éventuellement un trouble du rythme cardiaque.

Dans le cas de recueil de signaux d'ECG subcutané, les données collectées sont accumulées dans une mémoire de l'implant. Puis, pour permettre leur analyse, les données mémorisées sont transférées de l'implant à un dispositif externe ou "programmateur" permettant de vérifier le paramétrage du dispositif, de lire des informations enregistrées ou d'inscrire des données, ou encore de mettre à jour le logiciel interne de pilotage du dispositif.

Cet échange de données est effectué par télémétrie, c'est-à-dire par une technique de transmission d'informations à distance sans contact galvanique. Jusqu'à présent, la télémétrie était essentiellement réalisée par couplage magnétique entre des bobines du dispositif implanté et du programmateur, technique connue sous le nom de "procédé par induction",mais il a été récemment proposé de mettre en oeuvre une autre technique de couplage non galvanique utilisant les deux composantes d'une onde électromagnétique produite par des circuits émetteurs/récepteurs opérant dans le domaine des radiofréquences (RF), typiquement des fréquences de l'ordre de quelques centaines de mégahertz. Cette technique, dite de "télémétrie RF" permet de programmer ou interroger des implants à des distances supérieures à 3 m, et autorise donc l'échange d'informations sans manipulation d'une tête de télémétrie, et même sans intervention d'un opérateur externe.

Le US-A-5 331 966 décrit un implant pourvu d'électrodes de recueil de signaux d'ECG subcutané placées sur son boîtier, ainsi que de moyens de transmission RF, vers un dispositif externe, des données recueillies.

Les électrodes sont placées en partie sur la tête de connecteur et en partie sur le corps du boîtier proprement dit. En effet, pour un recueil satisfaisant les différentes électrodes doivent être éloignées le plus possible les unes des autres et disposées en une configuration sensiblement orthogonale. Le document propose également de disposer des électrodes sur la tranche du boîtier.

Cette dernière disposition est reprise par le US-A-6 522 915, qui décrit un élément support en forme de bracelet venant serrer l'implant sur sa tranche et portant à sa surface extérieure des électrodes de recueil en forme de fils spiralés. L'implant comporte par ailleurs une antenne de télémétrie RF, interne au boîtier ou disposée sur la tête de connecteur. Le US-A-6 631 290 décrit un configuration semblable, où les électrodes de recueil des signaux ECG sont situés sur la tranche périphérique de l'implant.

Les dispositifs décrits par ces antériorités présentent une structure relativement complexe et très spécifique, notamment pour tenir compte du placement des électrodes, qui est un point essentiel pour un recueil satisfaisant des signaux ECG.

Lorsque l'on souhaite équiper un modèle d'implant existant de fonctions de recueil d'ECG subcutané, ces contraintes imposent de redessiner le boîtier pour y incorporer les électrodes (comme dans les US-A-5 331 966 ou US-A-6 631 290), ou bien d'utiliser un élément rapporté dédié au recueil des signaux d'ECG (comme dans le US-A-6 522 915).

De plus, si l'on souhaite mettre en oeuvre une télémétrie RF, le placement de l'antenne par rapport au boîtier est également critique pour obtenir un diagramme de rayonnement satisfaisant, compte tenu du caractère conducteur du corps du boîtier - à la différence de la télémétrie inductive, qui est une technique robuste peu sensible à ces phénomènes.

C'est d'ailleurs pour cette raison que les antennes RF des dispositifs implantés sont généralement placées dans la tête de connecteur, car une antenne enfermée dans un corps de boîtier métallique ne pourrait pas rayonner de manière satisfaisante.

L'un des buts de l'invention est de proposer un perfectionnement aux dispositifs connus pourvus d'électrodes de recueil de signaux d'ECG subcutanés et de moyens de télémétrie RF, grâce à une structure d'électrodes de recueil rationalisée et qui en outre intègre une antenne RF.

L'invention a pour but de proposer une telle structure d'électrodes et d'antenne qui soit aisément adaptable aux dispositifs actuels en ce qui concerne les aspects matériels *(hardware),* en particulier qui n'exige pas une révision complète de la conception du boîtier.

On verra par ailleurs que la configuration de l'invention présente du point de vue technologique une structure relativement simple, donc avantageuse du point de vue des coûts de production, et qui est en outre polyvalente, c'est-à-dire adaptable aisément à différents types d'implants existants sans modification substantielle.

L'invention propose un dispositif implantable du type décrit notamment dans le US-A-5 331 966précité, correspondant au préambule de la revendication 1. L'invention comprend les éléments énoncés dans la partie caractérisante de cette revendication. Les sous-revendications visent des formes de réalisation particulières, avantageuses.

De façon caractéristique de l'invention, les électrodes ECG sont des électrodes surfaciques et l'antenne RF est une antenne surfacique. Le boîtier est un boîtier aplati présentant au moins une face sensiblement plane, et il comporte : une première région support, s'étendant sur la majeure partie de ladite face plane et recevant ladite pluralité d'électrodes ECG surfaciques avec une configuration où ces électrodes ECG sont sensiblement

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La figure 1 est une vue en élévation d'un dispositif selon une première forme de réalisation de l'invention, montrant les différentes électrodes de recueil de signaux d'ECG subcutané et l'antenne de télémétrie RF.
La figure 2 est une coupe schématique, non à l'échelle, selon la ligne II-II de la figure 1.
La figure 3 est une représentation montrant les divers blocs fonctionnels, reliés aux électrodes et à l'antenne, du dispositif selon l'invention.
Les figures 4 et 5 sont homologues des figures 1 et 2, pour une variante de réalisation de l'invention.
La figure 6 illustre une autre configuration encore des électrodes et de l'antenne du dispositif selon l'invention.

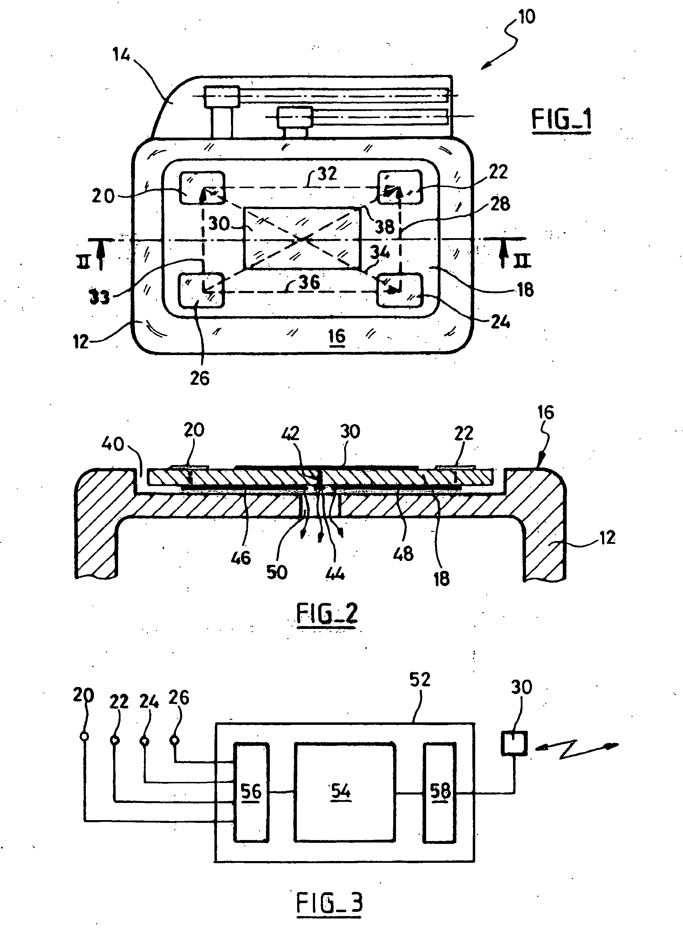

On va maintenant décrire un exemple de réalisation du dispositif de l'invention.
L'invention peut notamment être appliquée aux dispositifs implantables commercialisés par ELA Médical, Montrouge, France. Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. L'adaptation de ces appareils à la mise en oeuvre des fonctions de l'invention est à la portée de l'homme du métier, et elle ne sera pas décrite en détail.
Sur les dessins, la référence 10 désigne un dispositif implantable constitué d'un boîtier 12 contenant les divers circuits électroniques et la pile d'alimentation du dispositif, et surmonté d'une tête de connecteur 14 reliée mécaniquement et électriquement au boîtier et pourvue d'un ou plusieurs logements susceptibles de recevoir une ou plusieurs sondes de détection/stimulation endocavitaires implantées dans le myocarde.
Le boîtier 12 est un boîtier comportant au moins une face sensiblement plane 16 recevant, de manière caractéristique de l'invention, une plaquette support 18 portant des électrodes 20, 22, 24, 26 de recueil d'ECG subcutané ainsi que, dans ce mode de réalisation, une antenne de télémétrie RF 30.
Les électrodes ECG 20, 22, 24, 26 sont des électrodes surfaciques ou électrodes "patch", c'est-à-dire qu'elles sont constituées chacune d'une plage conductrice susceptible de venir en contact avec les tissus du patient à l'endroit où est implanté le dispositif. La forme de chacun de ces "patchs" n'est pas critique, elle peut être ronde, carrée, etc., le paramètre important étant, d'une part, l'aire du patch et, d'autre part, le fait que les diverses électrodes 20, 22, 24, 26 soient disposées dans un même plan.
Dans le mode de réalisation illustré figure 1, les électrodes ECG sont au nombre de quatre, et disposées sensiblement aux quatre coins de la plaquette 18, qui a elle-même une forme rectangulaire. La configuration des quatre électrodes est ainsi une configuration orthogonale, permettant de recueillir des signaux ECG selon une pluralité de vecteurs, jusqu'à six vecteurs 28, 32, 33, 34, 36 et 38. En plaçant ainsi les électrodes aux quatre angles de la plaquette 18, on maximise la distance séparant ces électrodes, ce qui est un facteur important pour un recueil satisfaisant des signaux ECG.
Le placement des électrodes sur la surface plate du boîtier selon une configuration sensiblement coplanaire permet de disposer celles-ci dans un plan sensiblement parallèle à la surface frontale du torse du patient, procurant des signaux proches de ceux typiquement fournis par des électrodes externes telles que celles habituellement utilisées avec un enregistreur externe Holter.
En variante, il est possible d'utiliser une configuration à trois électrodes seulement, la configuration orthogonale avec les électrodes 20, 22 et 24, permettant de recueillir des informations selon trois vecteurs 28, 32 et 34. Inversement, il serait possible de prévoir un nombre d'électrodes supérieur à quatre, mais au prix d'une complexité plus grande du traitement, dû à la multiplication du nombre de signaux ECG produits.
Quant à l'antenne RF 30, elle est également réalisée sous la forme d'un élément surfacique (antenne patch), qui peut être avantageusement situé dans la région centrale de la plaquette 18. Alors que la disposition relative des électrodes ECG est importante, le placement de l'antenne RF par rapport à ces électrodes ECG n'est pas critique (mais le placement de cette antenne RF est critique par rapport au boîtier, pour obtenir un diagramme de rayonnement satisfaisant, compte tenu du caractère conducteur du corps du boîtier). Cette antenne RF peut donc être située à un autre endroit de la plaquette, ou même sur une autre plaquette montée sur la face opposée du boîtier.

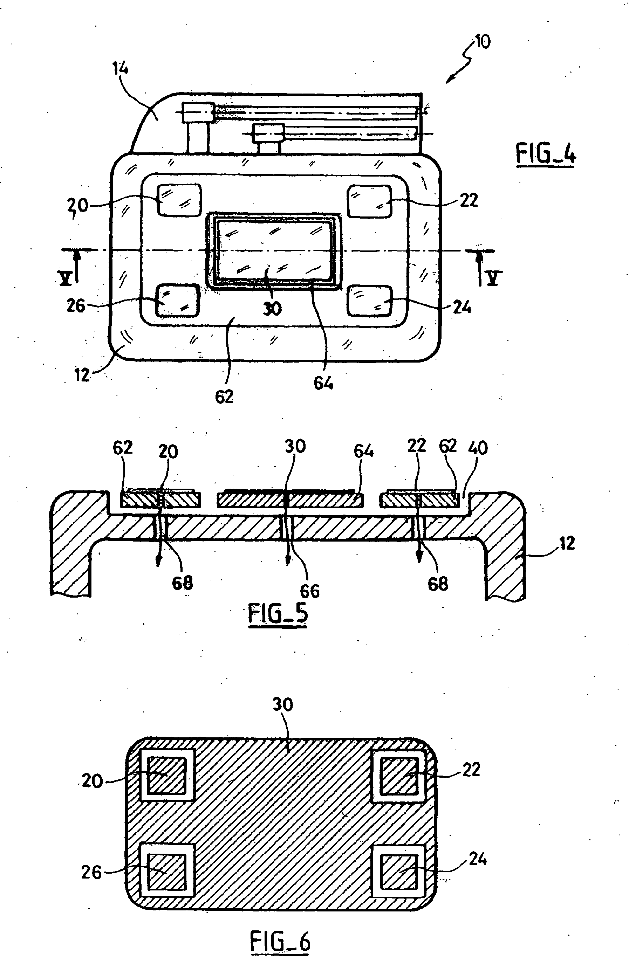

Concrètement, la plaquette 18 est constituée d'un substrat isolant, par exemple en alumine ou un composite alumine-ferrite-oxyde de titane. Sur la face libre de ce substrat sont déposées des métallisations, par exemple en platine ou en platine iridié, définissant les électrodes ECG 20, 22, 24, 26 et l'antenne RF 30. Très avantageusement, la plaquette 18 est disposée et collée au fond d'un lamage 40 formé dans la face plane du boîtier, de manière que la surface de la plaquette affleure la surface du boîtier sans discontinuité d'épaisseur.
La liaison des électrodes et de l'antenne aux circuits situés au sein du boîtier est assurée par des vias tels que 42 traversant l'épaisseur de la plaquette et débouchant sur la face opposée, où ils sont reliés à des conducteurs tels que 44, 46, 48 convergeant vers une traversée 50 formée dans l'épaisseur du boîtier, d'où ces conducteurs pourront être reliés aux divers circuits du dispositif.
Comme illustré schématiquement figure 3, les circuits 52 du boîtier comprennent, outre un module de traitement des signaux 54, un étage de recueil 56 relié aux électrodes 20, 22, 24 et 26 et un étage émetteur/récepteur RF 58 relié à l'antenne 30.
Le boîtier 12 peut être un boîtier conducteur (généralement en titane), ce qui ne crée pas de difficulté dans la mesure où les électrodes et l'antenne sont des éléments extérieurs au boîtier et isolés électriquement de celui-ci par le substrat céramique 18. Par ailleurs, le boîtier métallique 12 peut être éventuellement utilisé comme électrode indifférente pour le circuit de détection des signaux ECG.
Dans la variante illustrée figures 4 et 5, la plaquette commune 18 des figures 1 et 2 est remplacée par deux plaquettes support distinctes 62, 64, l'une 62 dévolue aux électrodes ECG 20, 22, 24, 26, l'autre à l'antenne RF 30. Pour conserver la même configuration d'ensemble que le mode de réalisation précédent, la plaquette 64 peut être logée dans un évidement central de la plaquette 62. Dans ce mode de réalisation, les électrodes et l'antenne sont reliées aux circuits internes du boîtier par des traversées spécifiques telles que 66, 68 formées au-dessous des éléments respectifs, électrodes ou antenne.
La figure 6 illustre une autre configuration possible des électrodes et de l'antenne du dispositif selon l'invention : la plaquette commune est principalement dévolue à l'antenne 30, qui s'étend ainsi pratiquement jusqu'aux bords du dispositif, tandis que les électrodes ECG 20, 22, 24, 26 sont formées par des îlots situés à proximité des quatre coins de l'antenne 30, mais en deçà du contour extérieur de cette dernière - à la différence par exemple de la configuration des figures 1 et 4, où les électrodes se situaient au-delà du contour extérieur de l'antenne 30.

Enfin, dans l'un ou l'autre mode de réalisation, le boîtier peut être également pourvu d'une bobine interne de télémétrie inductive, en complément de l'antenne surfacique externe de télémétrie RF.

## Revendications

1. Un dispositif médical implantable actif, notamment un dispositif de stimulation, resynchronisation, défibrillation et/ou cardioversion, comprenant un boîtier (12) de générateur présentant au moins.une face sensiblement plane, (16) et logeant les circuits électroniques du dispositif avec leur alimentation, dans lequel :
- le boîtier (12) comporte une première région support, s'étendant sur la majeure partie de ladite face plane (16) et recevant une pluralité d'électrodes ECG surfaciques (20, 22, 24, 26) pour le recueil de signaux d'ECG subcutané avec une configuration où ces électrodes ECG sont sensiblement coplanaires et disposées à distance les unes des autres, ces électrodes étant disposées sur une région extérieure du boîtier de manière à pouvoir venir en contact avec les tissus du patient environnant le boîtier après implantation de ce dernier ;
- le boîtier est en outre pourvu d'une antenne de télémétrie (30) ; et
- les circuits électroniques (52) comprennent un circuit (56) de traitement desdits signaux d'ECG subcutané, couplé auxdites électrodes portées par le boîtier, ainsi qu'un circuit (58) émetteur/récepteur de télémétrie, couplé à ladite antenne,
dispositif **caractérisé en ce que**:
- ladite télémétrie est une télémétrie RF non inductive et ladite antenne (30) est une antenne RF surfacique,
- le boîtier (12) comporte une deuxième région support, disposée en surface du boîtier, recevant l'antenne RF surfacique (30), et
- lesdites première et seconde régions support comprennent au moins une plaquette (18 ; 62, 64) rapportée sur ladite face plane du boîtier, avec un substrat isolant pourvu sur sa face libre de plages conductrices (20, 22, 24, 26; 30) formant respectivement lesdites électrodes ECG et l'antenne RF.

2. Le dispositif de la revendication 1, où lesdites première et seconde régions support sont toutes deux situées sur ladite face plane (16) du boîtier.

3. Le dispositif de la revendication 2, où la première région support est une région périphérique et la seconde région support est une région centrale de ladite face plane (16) du boîtier.

4. Le dispositif de la revendication 1, où ladite première région support est une région sensiblement en forme de rectangle et les électrodes ECG (20, 22, 24, 26) sont disposées au voisinage d'angles respectifs de ce rectangle.

5. Le dispositif de la revendication 1, où ladite plaquette (18) est une plaquette commune portant à la fois lesdites électrodes ECG et l'antenne RF.

6. Le dispositif de la revendication 1, où la plaquette (18) comprend des conducteurs de liaison (46, 48) sur sa face appliquée contre le boîtier, ainsi que des vias (42) reliant à ces conducteurs de liaison lesdites plages conductrices (20, 22, 24, 26, 30) formant les electrodes ECG et l'antenne RF.

7. Le dispositif de la revendication 6, où le boîtier (12) comprend une traversée (50) débouchant au droit d'un point situé sous la plaquette, et lesdits conducteurs de liaison (46, 46) convergent vers cette traversée.

8. Le dispositif de la revendication 1, où le boîtier (21) comprend sur ladite face plane(16) un lamage (40) de dimensions homologues de ladite au moins une plaquette (18 ; 62, 64), pour loger celle(s)-ci de manière affleurante.

## Claims

1. An active implantable medical device, in particular a device for pacing, resynchronization, defibrillation and/or cardioversion, comprising a generator casing (12) with at least one substantially planar face (16), and accommodating the electronic circuits of the device along with their power supply, wherein:
- the casing (12) includes a first supporting area, extending over the greater part of said planar face (16) and receiving a plurality of surface ECG electrodes (20, 22, 24, 26) for collecting subcutaneous ECG signals in an arrangement where said ECG electrodes are approximately coplanar and located at a distance from each other, said electrodes being located on an external area of the casing in order to enable contacting the patient's tissues surrounding the casing after implantation of the latter;
- the casing is further provided with a telemetry antenna (30); and
- the electronic circuits (52) comprise a circuit (56) for processing said subcutaneous ECG signals, coupled to said electrodes supported by the casing, along with a telemetry transmitter/receiver circuit (58) coupled to said antenna,
said device being **characterised in that**:
- said telemetry is a non-inductive, RF telemetry and said antenna (30) is a surface RF antenna;
- the casing (12) includes a second supporting area, arranged on the surface of the casing, receiving the surface RF antenna (30); and
- said first and second supporting areas include at least one board (18 ; 62, 64) added onto said planar face of the housing, provided with an insulating substrate having on its free face conducting pads (20, 22, 24, 26, 30) forming said respective ECG electrodes and said RF antenna.

2. The device of claim 1, wherein said first and second supporting areas are both located on said planar face (16) of the casing.

3. The device of claim 2, wherein said first supporting area is a peripheral area, and said second supporting area is a central area of said planar face (16) of the casing.

4. The device of claim 1, wherein said first supporting area is an area having approximately the shape of a rectangle, and the ECG electrodes(20, 22, 24, 26) are located in the vicinity of respective corners of said rectangle.

5. The device of claim 1, wherein said board (18) is a common board supporting both said ECG electrodes and said RF antenna.

6. The device of claim 1, wherein said board (18) includes connecting conductors (46, 48) on its face in contact with the casing, as well as vias (42) connecting said connecting conductors to said conductive pads (20, 22, 24, 26, 30) forming the ECG electrodes and the RF antenna.

7. The device of claim 6, wherein the casing (12) comprises a feedthrough (50) emerging at a point located under the board, and said connecting conductors (46, 46) converge towards said feedthrough.

8. The device of claim 1, wherein the casing (12) comprises on said planar face (16) a recess (40) which dimensions correspond to said at least one board (18; 62, 64) so as to accommodate the latter in a flush-mount manner.

## Patentansprüche

1. Aktive implantierbare medizinische Vorrichtung, insbesondere eine Stimulations-, Re-Synchronisation-, Defibrillierungs- und/oder Kardioversionsvorrichtung, umfassend ein Generatorkästchen (12), welches zumindest eine im Wesentlichen ebene Oberfläche (16) aufweist und die elektronischen Schaltkreise der Vorrichtung mit ihrer Versorgung aufnimmt, in welchem:
- das Kästchen (12) einen ersten Stützabschnitt umfasst, welcher sich auf dem Großteil der ebene Oberfläche (16) erstreckt und eine Vielzahl von ECG-Oberflächenelektroden (20, 22, 24, 26) aufnimmt, um subkutane ECG-Signale zu sammeln mit einer Konfiguration, wo diese ECG-Elektroden im Wesentlichen koplanar sind und mit einem Abstand zueinander angeordnet sind, wobei diese Elektroden auf einem äußeren Abschnitt des Kästchens in der Weise angeordnet sind, um in Kontakt mit dem Gewebe des Patienten zu kommen, welches das Kästchen nach der Implantation des Letzteren umgibt;
- das Kästchen unter anderem mit einer Telemetrieantenne (30) versehen ist; und
- die elektronischen Schaltkreise (52) einen Schaltkreis (56) zur Bearbeitung der subkutanen ECG-Signale, welcher mit den Elektroden gekoppelt ist, die von dem Kästchen getragen werden, genauso wie ein Telemetrie/Sender/Empfängerschaltkreis (58), welcher mit der Antenne gekoppelt ist, umfassen
welcher **dadurch gekennzeichnet ist, dass**:
- die Telemetrie eine nicht-induktive RF-Telcmetrie ist und die Antenne (30) eine Oberilächen-ItF-Antenne ist,
- das Kästchen (12) einen zweiten Stützabschnitt umfasst, welcher an der Oberfläche des Kästchens angeordnet ist, wobei er die Oberflächen-RF-Antenne (30) aufnimmt, und
- der erste und zweite Stützabschnitt zumindest eine Scheibe (18; 62, 64) umfasst, welche auf der ebenen Oberfläche des Kästchens angebracht ist, mit einem isolierenden Substrat, welches auf seiner freien Seite mit leitenden Bereichen (20, 22, 24, 26, 30) versehen ist, welche jeweils die ECG-Elektroden und die RF-Antenne bilden.

2. Vorrichtung nach Anspruch 1, wo die ersten und zweiten Stützbereiche beide auf der ebenen Oberfläche (16) des Kästchens angeordnet sind.

3. Vorrichtung nach Anspruch 2, wo der erste Stützbereich ein peripherer Bereich und der zweite Stützbereich ein zentraler Bereich der ebenen Oberfläche (16) des Kästchens ist.

4. Vorrichtung nach Anspruch 1, wo der erste Stützbereich ein Bereich ist, welcher im Wesentlichen rechteckige Form hat und die ECG-Elektroden (20, 22, 24, 26) in der Nachbarschaft der jeweiligen Ecken dieses Rechtecks angeordnet sind.

5. Vorrichtung nach Anspruch 1, wo die Platte (18) eine gemeinsame Platte ist, welche sowohl die ECG-Elektroden als auch die RF-Antenne trägt.

6. Vorrichtung nach Anspruch 1, wo die Platte (18) Verbindungsleiter (46, 48) auf ihrer gegen das Kästchen aufgebrachten Seite umfasst, genauso wie Bohrungen (42), welche die leitenden Bereiche (20, 22, 24, 26, 30) mit diesen Verbindungsleitern verbinden, welche die ECG-Elektroden und die RF-Antenne bilden.

7. Vorrichtung nach Anspruch 6, wo das Kästchen (12) ein Querstück (50) umfasst, welches direkt an einem Punkt heraustritt, welcher unter der Platte angeordnet ist und die Verbindungsleiter (46, 46) zu dieser Querverbindung zusammenlaufen.

8. Vorrichtung nach Anspruch 1, wo das Kästchen (21) auf der ebenen Oberfläche (16) einer Flachsenkung (40) homologer Abmessungen der zumindest einen Platte (18; 62, 64) umfasst, um diese in nivellierender Weise aufzunehmen.
